# EUROPEAN PATENT SPECIFICATION

(11) **EP 2 622 948 B1**
(45) Date of publication and mention of the grant of the patent: **24.08.2016**
(21) Application number: 11776223.7
(22) Date of filing: 22.09.2011
(51) Int. Cl.: H05H 1/24, A61C 17/02, A61B 18/00, A61L 2/14

(54) **ACTIVE GASES AND TREATMENT METHODS**
AKTIVE GASE UND BEHANDLUNGSVERFAHREN
GAZ ACTIFS ET PROCÉDÉS DE TRAITEMENT

(30) Priority: 28.09.2010 GB 201016341
(43) Date of publication of application: 07.08.2013
(73) Proprietor: Linde Aktiengesellschaft, 80331 München (DE)
(72) Inventor: LLOYD, Geoffrey Morgan, Sandhurst, GU47 0FP (GB); MASON, Rodney, Stewart, Blackpill, Swansea SA3 5BX (GB)
(74) Representative: Boult Wade Tennant
(86) International application number: PCT/GB2011/001383
(87) International publication number: WO 2012/042194

(56) References cited:
- EP-A2- 2 320 715
- WO-A1-2010/103262
- US-A1- 2004 045 806
- US-A1- 2008 142 057
- US-A1- 2009 004 620

## Description

### FIELD OF THE INVENTION

This invention relates to methods of forming active gases, and to the use of such gases in methods of treatment.

### BACKGROUND OF THE INVENTION

There is currently much research interest in the use of non-thermal gaseous plasmas in a number of therapeutic and oral care applications. Suggested uses of non-thermal gaseous plasma include the treatment of wounds, the cosmetic whitening of teeth, both to remove stains and to whiten tooth enamel, and the cleaning of teeth. See, for example, US-A-2009/004620 and EP-A- 2 160 081.

A non-thermal plasma is typically formed by striking an electric discharge between electrodes in a cell containing a helium atmosphere. Typically, a flow of helium passes through the cell and is then directed from the cell to a substrate to be treated. The effect of the electric discharge is to ionise some of the helium atoms in the cell. Other helium atoms are excited by the electric discharge. That is to say, in each excited helium atom, an electron is raised to a quantum level above its ground state. Excited and ionised helium atoms are no longer inert and can directly or indirectly mediate, for example, the sterilisation, at least in part, or the cleaning of a substrate or surface.

EP-A-2 160 081 seeks to improve the sterilisation effect of the plasma on, for example, wounds. That object is according to EP-A-2 160 081 achieved by mixing the helium with an additive. A large number of different additives are disclosed. Examples of gaseous additives include argon, krypton, xenon, nitric oxide, oxygen, hydrogen, sulfur hexafluoride, nitrous oxide, hexafluoroethane, methane, carbon fluoride, fluoroform, and carbon dioxide.

Water and ethanol are also disclosed as suitable additives. In order to form a mixture of a carrier gas (typically helium) and a gaseous additive, EP-A-2 160 801 discloses the use of separate sources of the carrier gas and the additive, and controlling their supply to a gas mixer which communicates with a plasma generator.

US 2008/0142057 discloses a cleaning device using atmospheric gas discharge plasma.

### SUMMARY OF THE INVENTION

According to one aspect of the present invention there is provided a method of making an active gas in accordance with claim 1.

The invention is based on a number of different findings in plasma chemistry. When non-thermal gaseous plasma is used in, for example, oral treatment, it is undesirable to generate the main discharge, which creates the primary plasma, in the oral cavity itself. It is therefore generated outside the oral cavity and plasma from it, flows into the oral cavity.

Strictly speaking, once the flow of gas mixture leaves the plasma generator (i.e. the main discharge), it may no longer be in the form of a plasma unless an external electrical potential, of sufficiently high value to create a subsidiary current (but much smaller than the main discharge) continues to be applied to it, since without a current flow through it, it rapidly decays. It is simply an active gas mixture containing ionic, excited and reactive neutral species.

The term "plasma" shall be reserved herein for the description of a partially ionised gas or gas mixture to which an electric potential is applied, and through which a small electric current flows. The plasma typically glows. The flow of the gas mixture along the applicator shall be referred to as an "afterglow", and the flow of the gas mixture once it has exited the applicator shall be referred to as a "plume". The term "more readily ionisable gas" shall be reserved herein for the description of a gas which is more readily ionisable (i.e. takes less energy to be ionised) than helium.

The plume is exposed to the water vapour. The active gas mixture flows from the generator though a passage and then is ejected from the passage into a standing gaseous atmosphere. The passage may be for example a tube or similar applicator, which tube expels gas from the downstream end into the atmosphere. If desired, the downstream end of the tube can be inserted into the oral cavity and pointed at the surface to be treated.

The standing gaseous atmosphere contains water vapour and flow of the gas mixture through the gaseous atmosphere thereby exposes the gas mixture to the water vapour. The standing gaseous atmosphere is ambient air. The gas mixture may be exposed to water vapour by humidifying at any convenient point downstream from the generator. Other methods of exposure are described herein, such as where the gas mixture may be exposed to water vapour in the passage.

The gas pressure in the generator is preferably in the range of from 0.5 to 2 bar, more preferably in the range of from 0.9 to 1.1 bar. In other words, the generator is preferably operated at atmospheric pressure. At such pressures, the proportion of the more readily ionisable gas in the gas mixture can be less than 2% by volume.

If the gas supplied to the plasma generator is essentially pure helium, the resulting plume may contain relatively few ions. It is to be understood that the number of ions in the active gas mixture of a plasma is usually related to the number of other active species, namely free radicals and excited atoms and molecules also created. Accordingly, it is to be expected that the helium plume will be a less effective treatment agent, for example an oral treatment agent.

The most stable low pressure glow discharges are with the inert gases. This is because they are atomic, their rate of recombination (X⁺ + e⁻ → X) is very slow, and exchange of energy is all electronic. This compares with all molecular gases which recombine very quickly and exchange energy very quickly leading to quenching using vibrational and rotational energy modes. Using Kr or Xe can be advantageous. These gases are however rare and expensive. Preferably helium and argon are used.

By exposing the flow of the gas mixture to a source of water vapour, the population of ions and beneficial active species, such as OH, in the plume is believed to greatly increase. It is therefore expected that the resulting plume will be a more effective treatment agent, for example an oral treatment agent.

The exposure is affected simply by passing the plume through an atmosphere containing water vapour, namely, normal atmospheric air. Other methods of exposure are described herein, such as where the exposure to the water vapour is affected by introducing water vapour (for example, carried in a noble gas stream) into the plume or afterglow. Alternatively, it is also described that water vapour is premixed with the carrier gas, for example, a mixture of carrier gas, more readily ionisable gas and water vapour may be supplied from a pressure vessel that has been prefilled with such mixture. An advantage of affecting the exposure by passing the plume through an atmosphere containing water vapour is that it avoids the need to prepare a gas mixture or gas stream carrying water upstream of the exposure.

The flow of gas mixture may be exposed to a concentration of at least 1 ppm of water vapour. Our experiments have included those with as much as 330ppm of water vapour. Typical exposures are at a concentration between about 20 to about 100 ppm of water vapour, and particularly to between about 40 to about 80ppm, and more particularly to between about 50 to about 60 ppm of water vapour.

It has however been found that there is no simple linear relationship between the population of excited hydroxyl radicals (which can be detected by their emission spectrum) in the plume and the concentration of water vapour the carrier gas and ionisable gas are exposed to.

There is, we believe, another useful effect of making an active gas according to the method of the invention. Our experiments have shown that the emission spectra of an active gas mixture of helium which has been exposed to water vapour has a strong peak at 777nm indicating the presence of excited oxygen atoms (singlet oxygen). This also may be an indication (but not necessarily) of the relative concentration of ground state atoms. This singlet oxygen peak is visible in the plasma generator. Both types of oxygen atom will inevitably with molecular oxygen form ozone. Exposure to too much ozone causes negative side effects to patients and also causes damage to the environment. Our experiments have shown that the intensity of the singlet oxygen peak at 777 nm in the plume is significantly reduced when the active gas is prepared according to the method of the invention. As a result, the subsequent formation of ozone appears also to be suppressed. As is supported by the absence of an odour of ozone. This is an advantage of the invention as any negative side effects experienced by the patient will be reduced.

It is believed on the basis of our observations that the strength of the excited OH intensity peak (at 308 nm) and therefore probably the concentration of OH radicals in the plume having been exposed to water vapour is dependent on the flow rate of the gas mixture.

In order to optimise the population of ionic species in the plume, the method according to the invention may be adapted so that the gas mixture flows through the generator at a rate of 5 L/min or less, preferably at a rate of 2 L/min or less, more preferably at a rate of 1 L/min or less, for example at a rate of 0.5 L/min or less.

The plume temperature has also been observed to be dependent on the flow rate of the gas mixture. The flow rate of the gas mixture therefore has to be selected in order to balance the need to optimise the population of desirable species (e.g. OH) and the need to have a desirable plume temperature, i.e. one below 42° C.

According to a further aspect of the present invention there is provided a method of non-clinical or cosmetic oral treatment, comprising making an active gas in accordance with the method of claim 1, and causing the flow of the active gas downstream to perform the non-clinical or cosmetic oral treatment.

The non-clinical or cosmetic oral treatment may comprise:
- the removal of stains from teeth;
- the whitening of tooth enamel;
- the general cleaning of teeth to destroy harmful bacteria;
- the interdental cleaning of teeth;
- the freshening of breath;
- the treatment of halitosis
- the in *situ* cleaning of orthodontic braces
- the *in situ* cleaning of dental implants.

In each of the above examples, the flow of the gas mixture downstream of the plasma generator may be directed at the tooth or teeth to be treated, or the area of gums to be treated, or in the case of breath freshening or treatment of halitosis, at the back of the mouth for a sufficient period of time to have a desired effect.

Normal treatment time periods for a typical treatment are from ten seconds to ten minutes. The treatment may be repeated daily or at shorter or longer intervals.

The active gas preferably has bactericidal activity, for example oral bactericidal activity. Preferably the active gas has tooth whitening activity.

It will readily be appreciated that the principles of the invention may be applied to clinical oral care treatment.

According to a further aspect, there is provided the use of an active gas prepared according to the method of the invention for bactericidal and/or whitening activities. This can include use for cleaning items such as for example laundry or for dishwashing purposes.

According to a yet further aspect of the invention, there is provided a method of stain and/or bacteria removal, comprising: making an active gas in accordance with the method of claim 1; and directing a flow of the active gas at an article.

The method may be utilised for stain removal and/or bacteria removal on either biological items, such as for example teeth or other parts of human or animal bodies (for example, in wound treatment) or non-biological items such as dishwashing items or laundry items. Laundry items may typically comprise textiles or clothing. Dishwashing items may typically comprise eating or cooking utensil.

The plasma generator has a gas outlet temperature of from 5° C. to 42° C., for example from 10° C. to 40° C. Higher gas temperatures are generally unsuitable for oral treatments and may damage the mouth or teeth if sustained for too long a period. Temperatures lower than 5° C. may be found uncomfortable by the person undergoing the treatment. The desired gas outlet temperature of from 5° C. may typically be achieved without cooling at argon concentrations below 2% by volume. If cooling is needed, it may, for example, be applied thermo-electrically to a heat conductive thermal mass in thermal contact with the plasma generator.

It has been found that the temperature of the plume is dependent on the amount of energy deposited into the plasma and thus weakly on the amount of more readily ionisable gas (for example argon) present within the gas mixture. The temperature of the plume increases as the amount of more readily ionisable gas in the gas mixture increases. As discussed above, the plume temperature is preferred to be 42 °C or less. In accordance with our experimental findings, the amount of more readily ionisable gas is preferably less than about 30%, preferably less than 25%, and in the case of argon more preferably less than about 4%, for example less than about 3%.

If the amount of more readily ionisable gas is greater than about 4% by volume cooling, for example by thermo-electric means, can be provided to limit any temperature rise of the plume in order to ensure that the plume temperature does not exceed about 42°C.

We have also found that the population of excited hydroxyl radicals (observable by their emission at 308 nm) in the plume may be dependent on the concentration of the more readily ionisable gas (for example argon) present in the gas mixture. There does not however appear to be a simple linear relationship between the population of excited hydroxyl radicals or ionic species in the plume and the concentration of water vapour downstream of the generator. We attribute these results partly to a tendency we have found for a molecular additive gas to quench the non-thermal plasma in the plasma generator. Once the maximum is reached, the plasma-quenching effect reduces the total number of ions present in the plume. As helium has a particularly high ionisation energy, ions of the additive gas will be formed preferentially in the discharge, making possible the use of concentrations of argon of less than 2% by volume when, for example, the plasma is generated at atmospheric pressure.

When the concentration of the more readily ionisable gas increases beyond a certain point our experiments have shown that the glow discharge is lost and arcing is seen. This is undesirable as the temperature of the plume increases and becomes unsafe and uncontrollable. From an oral point of view, it is desirable to have a maximum plume temperature of 42 °C in order to prevent any damage to the tooth pulp from prolonged exposure.

In order to obtain a desirable gas outlet temperature together with a high population of active species in the plume the more readily ionisable gas, for example argon, may be present in an amount of between about 0.01 and about 40% by volume, preferably between about 0.01 and 30%, more preferably between about 0.01 to about 25% by volume, for example between 0.01 and 20% by volume.

For example, there can be provided a prepackaged gas mixture kit comprising at least one pressure vessel comprising the carrier gas (e.g. helium) and the more readily ionisable gas (for example argon). The prepackaged gas mixture kit may further include a second vessel comprising water vapour. In some examples, the water vapour is retained on or in a substrate in a closed vessel. The plasma generator and the closed vessel may have configurations such that when the vessel is inserted in the plasma generator the closed vessel may be pierced or punctured in such a way that a gas path through the vessel is created, enabling gas to be charged with water vapour.

In a further aspect, the invention provides a handheld oral hygiene device comprising an apparatus for making an active gas according to the invention. The handheld device may contain a battery. The handheld device may contain a gas compartment, which may typically contain a mixture of the carrier gas and the more readily ionisable gas, and, optionally, water vapour. Optionally a further gas compartment may be provided containing water vapour.

The handheld device may be a toothbrush which would typically further comprise bristles.

The plasma generator preferably comprises a housing, at least one cathode and at least one anode, and a voltage signal generator operatively associated with the cathode and the anode. The voltage signal generator preferably generates a pulsed DC or an AC or an RF voltage signal suitable for the generation of a non-thermal plasma. It is possible to transform a low DC voltage in the order of 5 to 15V into a suitable AC or pulsed DC voltage to provide a glow discharge in the plasma generator.

The plasma generator, a battery for generating a DC voltage and electrical circuitry for transforming the DC voltage in a non-thermal plasma generating voltage may all be located in the same housing. The housing may have a configuration which enables it to be held and operated in the hand. The housing may also contain or dock a pressure vessel in the form of a capsule for storing the gas mixture. The capsule may have a (water) capacity of from 10 to 100, preferably 10 to 40ml, and, when full, a pressure of at least 50 bar, preferably at least 100 bar.

The partially ionised gas may flow out of the plasma generator directly to the atmosphere. Alternatively, it may flow from the plasma generator to an applicator. Although it is often convenient to have a tube or other applicator that can readily be pointed at the location to which the partially-ionised gas is to be directed, it is desirable to keep the length of the applicator to a minimum and typically less than 5cm or 10cm. This is because ionic, excited or reactive species in the partially-ionised gas mixtures tend with time to revert to their less active ground state or a benign non-reactive reaction product. The applicator has the advantageous effect of preventing or inhibiting back entrainment of air into the discharge. Such back entrainment would give rise to ozone and NOₓ which are potentially hazardous, would reduce the efficiency of the plasma generator, and would make its output unpredictable.

### BRIEF DESCRIPTION OF THE DRAWINGS

The methods according to the invention will now be described, by way of example, with reference to the accompanying drawings, in which
Figure 1 is a schematic diagram of an experimental non-thermal gaseous plasma generating apparatus which may be used to perform methods in accordance with the invention.
Figures 2A and 2B are images of an electric toothbrush head which has been dismantled.
Figure 3 is an image of a plume passing through the toothbrush head of Figures 2A and 2B, through a right angle when drawn to a metal object.
Figure 4 shows emission spectra of plumes resulting from the partial ionisation of (a) 5000 ppm Ar/He; (b) helium; (c) 10 ppm H₂O/He; (d) 250 ppm H₂O/He; and (e) 500 ppm H₂O/He.
Figures 5 and 6 are graphs of OH peak intensity at 308nm of plumes with respect to water concentration (ppm). The plumes resulting from the partial ionisation of a gas mixture of 5000 ppm Ar/He after being exposed to water vapour within the plasma generator and after exposing the plume to water vapour.
Figure 7 is a graph of excited OH peak intensity at 308 nm with respect to water concentration (ppm) of a plume resulting from a gas mixture of 5000ppm Ar/He. The graph shows the results for a flow rates of 2 L/min and 1 L/min
Figure 8 is an optical emission spectrum of plumes resulting from the partial ionisation of 5000 ppm Ar/He at four different flow rates (2L/min, 1.5 L/min, 1 L/min and 0.5 L/min).
Figure 9 is an optical emission spectrum of plumes resulting from the partial ionisation of a gas mixture of 330 ppm H₂O/Ar diluted with He at four different flow rates (2L/min, 1.5 L/min, 1 L/min and 0.5 L/min).
Figures 10A & 10B are graphs of excited OH peak intensity at 308 nm with respect to water concentration (ppm) of a plume resulting from a gas mixture of 330 ppm H₂O/Ar diluted with He.
Figure 11 is a graph of plume temperature measured 2mm from plume exit with respect to the Ar concentration in the gas mixture.
Figure 12 is a graph for comparative purposes illustrating the bactericidal effect over time of a plume resulting from the partial ionisation of a gas mixture of 5000ppm Ar/He (flow rate 0.5 min).
Figures 13 and 14 are sets of photographs for comparative purposes illustrating the effect on *Streptococci mutans bacteria* of a plume resulting from the partial ionisation of a gas mixture of 5000ppm Ar/He at 2mm from plume exit. The gas flow rate is 0.5L/min and the bacterial colonies have been exposed to the plume for 1 minute.
Figure 15 illustrates the whitening effect over time of the plume resulting from the partial ionisation of a gas mixture of 330ppm H₂O/Ar diluted with He (∼1:5) at a distance of 2mm.
Figure 16 is the whitening effect over time of the resulting plume from the partial ionisation of a gas mixture of 5000 ppm Ar/He at a distance of 2 mm.
Figure 17 illustrates the whitening effect over time of the plume resulting from the partial ionisation of a gas mixture of 330ppm H₂O/Ar diluted with He (∼1:5) at a distance of 10mm for 45 minutes and a distance of 2mm thereafter.
Figure 18 illustrates schematically the experimental apparatus employed in the Examples below.

### DETAILED DESCRIPTION

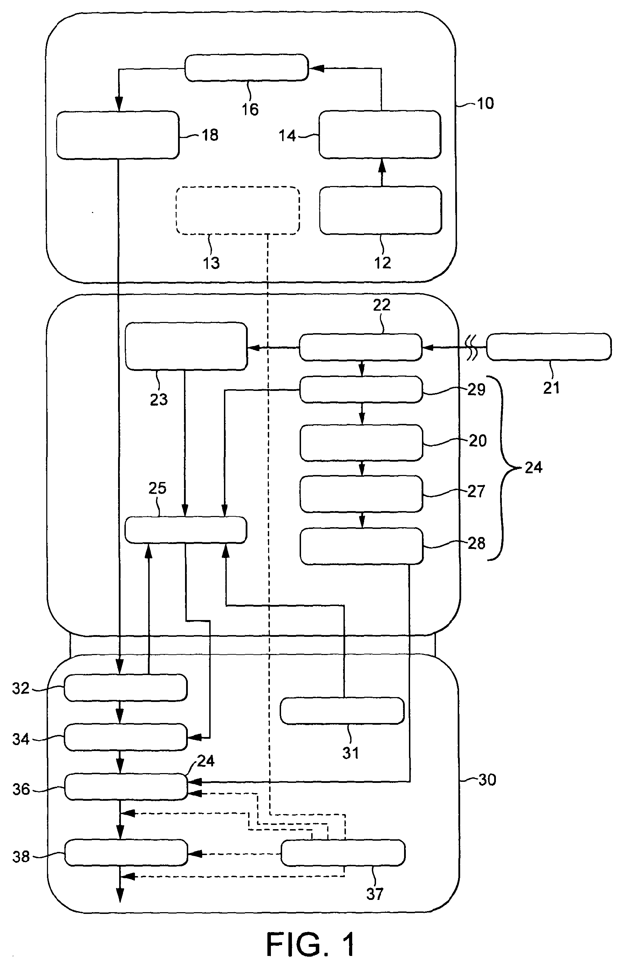

Referring to Figure 1, apparatus is shown for generating non-thermal plasma. The plasma may be in the form of a gas plume emitted from the apparatus. In the embodiment illustrated, the apparatus includes a gas supply 10, a handheld unit (or first housing) 30 and a bench unit 20 (or second housing). The apparatus provides a flow of a modified gaseous species for treatment of a region of a human or animal body. A generator 36 having at least one anode and one cathode which can be energised for forming a non-thermal plasma is located in the handheld unit 30. The handheld unit 30 has a configuration which enables it to be held by hand and operated for treating the treatment region. An inlet port is provided in the housing for a flow gas communicating with the generator and an outlet port allows flow of the modified gaseous species from the generator. The bench unit 20 is remote from the first housing, and has located in, for example, a battery compartment one or more batteries and a signal generator for converting voltage generated by the battery when located in the second housing into a pulsed high voltage signal. A gas passage through the second housing is connectable to the inlet port by a hose having a gas conduit and electrical leads for applying the pulsed high voltage signal to the said at least one electrode of the generator.

The gas cylinder 12 is provided as the pressure vessel of the gas supply. This is typically a 1 litre or 1.5 litre cylinder filled with helium and an additive gas, suitable for generating non-thermal plasma for providing a beneficial or therapeutic effect on a treatment region of a human or animal. In this way, the modified gas stream may, for example, include hydroxyl radicals (OH) and metastable atoms which are effective for stain removal from teeth. It will be understood that the cylinder is not limited to the size or content indicated although the upper limit is preferably less than 1.5 litres so that the apparatus is portable by hand. The cylinder may be provided with an integral pressure regulator 14. Located external to the cylinder 12 is a flow controller 16. The flow controller 16 may be set to a value in the range of 0.5 to 5l/minute. A further pressure regulator 18 is located downstream of the flow controller 16. This pressure regulator 18 releases gas flow at a pressure of 1.5 bar absolute. An outlet port of the gas supply is connected to an inlet port of the bench unit 20 by a hose. The gas supply 10 may optionally also contain a cylinder 13 (shown in hashed lines in Figure 1) for the supply of water vapour. The flow controller 16 may control the flow from the cylinder 13 or the cylinder may be provided with an independent controller (not shown).

The bench unit 20 includes a battery 22 and energising means 24 electrically connected to the battery 22. The battery illustrated is a 12V battery. The battery may be supplied with a battery charger 21 and a display LED 23. The display LED can indicate the status of the battery, i.e. it informs the user when the available power in the battery 22 is low. The bench unit comprises means for locating the battery or batteries in the unit so that they are placed in contact with electrical connections or terminals. The locating means may comprise a battery compartment shaped to receive the required battery or batteries and a removable cover for closing the compartment. The bench unit further comprises a gas passage for conveying gas from the gas supply 10 to the handheld unit 30.

The energising means as described in more detail below comprises one or more signal generators which receive energy from the battery and one or more electrodes driven by the generators for energising a plasma in a plasma generator. The energising means may include a low voltage signal generator 27 in connection with a high voltage generator 28.

In the illustrated arrangement, the signal generators convert the electrical current from a 12V battery into a high frequency AC output voltage in the range <1 to 6kV (0 to peak) at a frequency of 2-100 kHz which is suitable for generation of a non-thermal plasma. A transformer can be used to step up the voltage and enable voltages in the desired range of 3 to 6kV (0 to peak) to be generated. DC high voltage pulses may also be generated. In order to produce clear, well defined pulses it is desirable to keep the number of turns and inductance of the windings of the transformer to low levels and to have modest step-up ratios. This approach helps keep the unwanted parasitic elements of leakage inductance and stray winding capacitance to a minimum.

Because a pulse transformer has low primary winding inductance, the magnetising current that generates the working magnetic flux in the core is substantial, leading to significant stored magnetic energy in the transformer during the pulse record. For an efficient design, this magnetic energy is recovered at the end of the pulse and temporarily held in another form (usually as a charge on a capacitor) ready to generate the next pulse.

The magnetic flux in the core of the transformer must be returned to zero before the next pulse is generated otherwise the flux builds up with successive pulses until the core saturates, at which point the transformer stops working and acts as a short circuit to the drive electronics.
A common method of magnetic energy recovery in switched-node power supply transformers, which may be used in this case, is through the use of a so-called "flyback" winding. This is usually identical to the primary winding and both are wound on the core at the same time (a bipolar winding) in order to ensure a high level of magnetic coupling between the two. The flyback winding connects between ground and the reservoir capacitor of the DC supply via a blocking diode.

During pulse generation a fixed voltage is applied to the primary winding and current ramps up building up magnetic flux in the core - this induces an equal and opposite voltage across the flyback winding (but no current flows due to a blocking diode). Interruption of the primary current at the end of the pulse forces the magnetic field to start collapsing which reverses the induced voltage across the flyback winding and causes current to flow back into the supply capacitor. The flux and current ramp down smoothly to zero ready for the next pulse.

Another suitable transformer configuration is a push-pull design in which two identical bifilar wound primary windings are alternately connected to the DC power supply. The phasing of the windings is such that magnetic flux in the core is generated with opposing directions which each is alternately driven.

A push-pull design also allows stored magnetic energy to be recovered and returned to the supply capacitor in a very similar fashion to the flyback approach, where the blocking diode now becomes an active transistor switch. The same transformer design may be used for either approach. Although the push-pull design requires additional switching transistor and control, it allows the possibility of doubling the change in magnetic flux within the limits of the core by using both positive and negative flux excursions. The flyback design outlined above only allows unipolar flux excursions. For a given flux ramp rate, the push-pull design has the capability continuously to produce a continuous pulse with twice the duration of a flyback version using the same transformer.

A control 25, which may be in the form of a logic circuit is configured to receive a plurality of inputs which are dependent on a condition of the apparatus and selectively supply an output to the energising means, for example signal generator 27 in this example, for energising a plasma in the plasma generator 36. The control is electrically connected to the switch 31, flow sensor 32 and valve 34 by an electrical cable running through flexible hose. The signal generator may be configured to generate a signal for driving the electrodes which may be a low duty cycle signal in which the energy is provided to the or each electrode for less than 15% of the cycle.

In the embodiment illustrated in Figure 1 the power source is a 10 kV high frequency AC driver. It is able to generate high voltage trains, pulsed on for between one and ten milliseconds and off for between one and ten milliseconds respectively, thus giving rise to a 'mark (voltage on) to space (time between leading edge of adjacent voltage trains' ratio variable between 0.1 and 0.9. Alternatively, a continuous high frequency high voltage can be applied when the power applied can be partially controlled by varying the size of the peak to peak voltage applied.

The handheld unit 30 is configured by, for example, size and shape and weight such that it can be operated by a user and the resulting plasma easily directed by the user to treat an oral region of a human or animal body. The handheld unit 30 includes a flow sensor 32, a solenoid valve 34, a reaction generator 36 and a nozzle 38. The solenoid valve is located downstream of the flow sensor 32. The reaction generator is located downstream of the solenoid valve 34. The reaction generator is provided with a plurality of electrodes therein. The nozzle 38 is located downstream of the generator 36 and is adapted for directing a plasma plume to a treatment region of a human or animal. Also located within the unit 30 is an on/off switch 31.

The sources of water vapour 13 and 37 may be cylinders of noble gas charged with the requisite amount of water vapour (not within the scope of the invention).

The flow of gas is exposed to a source of water vapour 37 downstream of the nozzle (in the plume). Other methods of exposure are described herein, such as where the flow of gas is exposed to a source of water vapour 37 either at the reaction generator 36 or downstream of the generator 36. As shown by the hashed lines in Figure 1, the source of flow of gas may be exposed to the water vapour, in the generator 36, downstream of the generator (in the after glow), or at the nozzle 38.

A method of operating the apparatus will now be described with reference to Figure 1.

The control 25 is configured to receive inputs from low battery monitor 23, switches 29 and 31, and the flow sensor 32. The control outputs to the signal generator 27 and the solenoid valve 34 when prescribed inputs are received by the control. In this regard, the low battery monitor 23 sends a signal to the control indicating whether or not there is sufficient energy stored in the source 22 for energising plasma in the plasma generator 26. If the source has insufficient energy the control does not allow operation of the apparatus. If the source 22 has sufficient energy, the apparatus can be activated by operation of desk top switch 29 which outputs a signal to the control 25. The hand unit switch 31 is subsequently operated to supply an output to the control 25. The control receives the output from the switch 31 and if the output is positive and provided the switch 29 has been previously operated, the control sends an output to the solenoid valve 34 causing it to open to allow the flow of gas from pressure regulator 18. The flow sensor 32 sends an output to the control 25 if it senses that the flow of gas into the plasma generator 26 is above a predetermined mass or volume flow rate. If the control receives a positive output from the sensor 32, the control emits a control signal to the energising means 24 thereby allowing the low voltage signal generator to be energised together with the high voltage generator for activating the electrodes. Accordingly, a plasma can be energised in the plasma generator only if gas flow through the generator is above a predetermined rate.

During operation of the device gas flowing from the pressure regulator 18 passes through the flow sensor 32. The gas flow then continues to pass through the solenoid valve 34 to the reaction generator 36. If the flow rate is the required amount, as predetermined, the electrodes in the reaction generator are activated by the signal generator as part of the energising means 24. The gas is thus energised and forms a gas plasma. The plasma exits the generator 36 and passes through the nozzle 38. The flow of gas is exposed to a source of water vapour 37 downstream of the nozzle (in the plume). Other methods of exposure are described herein, such as where the flow of gas is exposed to a source of water vapour 37 in the generator 36, downstream of the generator (in the after glow), or at the nozzle 38. The nozzle 38 increases the velocity of the flow of plasma. The user directs the nozzle 38 towards the treatment area. The nozzle 38 may be replaceable.

As will be appreciated by the person skilled in the art, the apparatus of claim 1 may be provided in a compact form suitable for use as a handheld oral hygiene device. For example the apparatus may be provided in the form of a rechargeable electric toothbrush with a replaceable or replenishable gas supply.

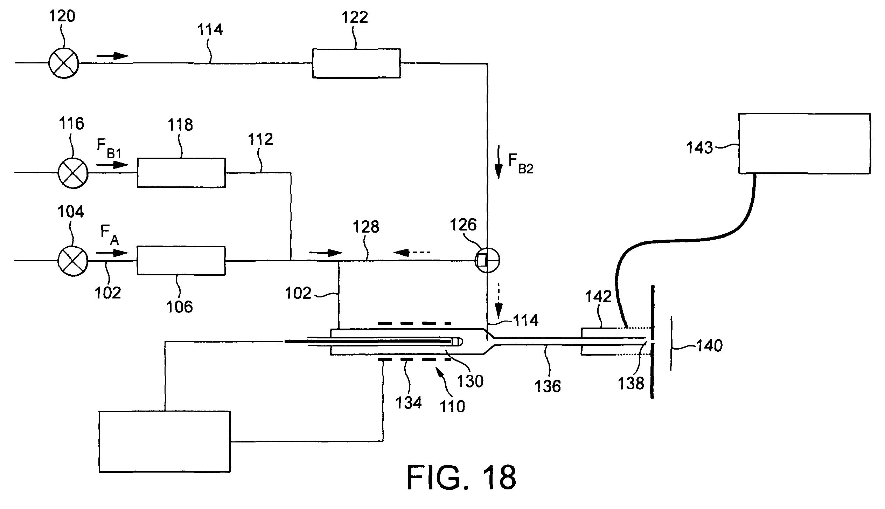

Referring now to FIG. 18 there is shown the apparatus used to perform the Experiments described below. This apparatus is similar in basic operating principle to that shown in Figure 1, but has a number of unique features that will particularly be described below.

The experimental apparatus comprised a main pipeline 102 for carrier gas, the main pipeline 102 having disposed therein a stop valve 104 and a mass flow controller 106 to set the mass flow rate. The main pipeline 102 terminates in a non-thermal plasma generator 110. There are two additional pipelines 112 and 114 for adding the more readily ionisable gas (argon) and the additive gas (water vapour). The pipeline 112 has a stop valve 116 and a mass flow controller 118 disposed therealong. The pipeline 112 terminates in a region of the pipeline 102 upstream of the plasma generator. The pipeline 114 has a stop valve 120 and a mass flow controller 122 disposed therein. The pipeline 114 terminates in an outlet from the plasma generator 110, the arrangement being such that gas added via the pipeline 114 does not influence the flow discharge. A further valve 126 is located in the pipeline 114. This valve is operable to place the pipeline 114 in communication with the pipeline 102 via a conduit 128. Thus the pipeline 114 is able to place either the plasma generator or the afterglow in communication with a chosen source of gas.

The plasma generator 110 comprises a tubular chamber 130 with an internal electrode 132 and an external electrode 134 connected to a power supply 136 and also grounded to Earth. The tubular chamber 130 is connected to or integral with an applicator tube 136 which terminates in a nozzle 138. The nozzle 138 terminates at a selected distance either 2mm or 10mm from a target 140 and may have a simple tubular form.

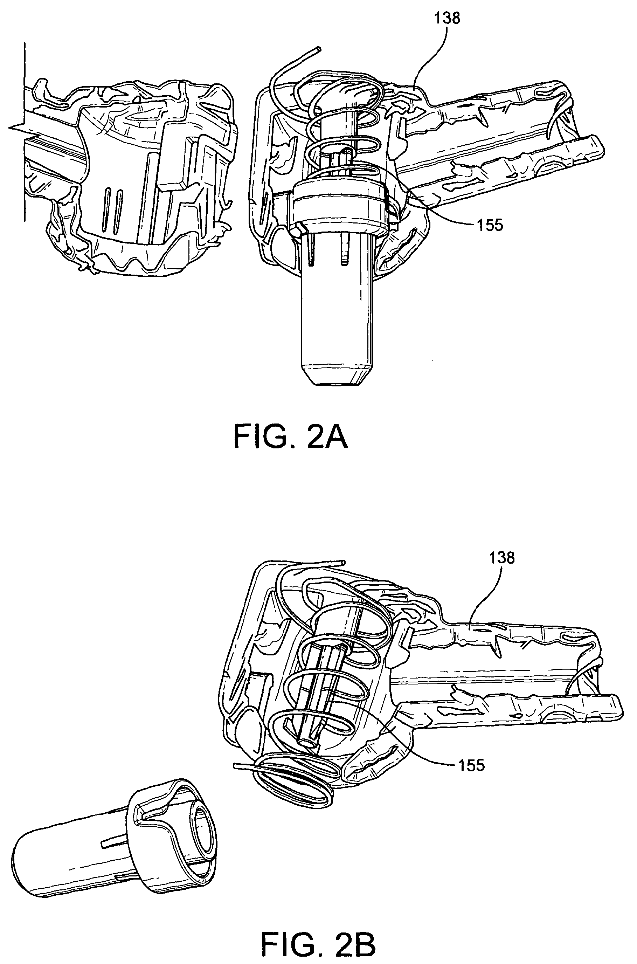

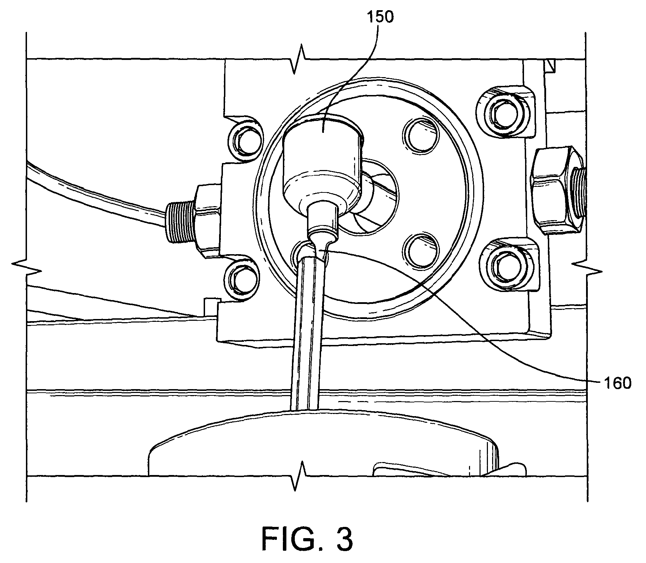

By way of illustration, one alternative form of nozzle 138 is better shown in Figures 2A, 2B and 3. As shown in Figures 2A and 2B, the nozzle is a commercial device intended for water. It has a spigot tool 155. In our experiments, this spigot 155 was omitted. In Figure 3, there is shown a glowing plume 160 being ejected from the nozzle 138, at right angles to the upstream flow. This plume 160 is exposed to the ambient air which will typically carry water vapour. The applicator nozzle is preferably constructed from or lined internally with PTFE, FEP or PFA or other plastic or other material which is non-reactive with the active plasma species. Preferably the construction material or lining is PFA.

Referring again to Figure 18, the experimental apparatus is provided with an
outlet chamber 142 arranged so as to allow emission spectroscopic measurements of the plume to be made by an optical emission spectrometer 143.
The experimental apparatus employed a plasma generator 110 in the form of a tubular quartz discharge tube 130 of 4mm internal diameter which is connected to a narrower quartz applicator tube 136 (30mm in length, and 2.5mm in internal diameter). The cylindrical outer electrode 134 was made using silver paint on the discharge tube and using metallised (silver loaded) epoxy resin to bond to an earth cable. The inner electrode 132 was formed of copper wire strands inserted into a narrow quartz tube, closed at one end, and packed with graphite to make uninterrupted contact between the rod and the inner surface. In another embodiment the inner electrode tube was coated in conducting silver epoxy resin. This was inserted into the discharge tube130 to make a concentric double dielectric electrode discharge. The quartz wall thickness of the inner electrode was 0.6mm and the outer electrode 0.9mm. The gap between the electrodes was approximately 0.4mm. The discharge region was about 15mm long. The arrangement was held together by machined parts of PEEK and O-rings of EPDM were used to seal the various parts to each other. Gases were supplied through stainless steel pipe and Swagelock fittings. These choices of material were made so as to keep down gas entrainment from the atmosphere and general gas desorption.

The choice of materials described above was made with a view to minimising the effect of outgassing on the gas composition to be subjected to the electric discharge.

In the experiments described below the discharge generating non-thermal gaseous plasma was powered by a high frequency AC (67 kHz) circuit operating at up to 7kV (peak to peak), which was gated on and off at 77 kHz in order to control the average power feed into the discharge. The power supply was driven by a DC supply operating at 10V. The current drain depended linearly on the fraction of time for which the output high frequency was gated on. The maximum DC current was set at less than 1A. The range of current drawn was between about 0.15 and 1.0A, depending on the composition of gas in the discharge and the gating ratio. At low current consumption (0.15A), the AC voltage pulses endured for approximately 1 ms (the so-called "mark") with the gap between voltage pulses being in the range of 12 ms (the so-called "space"). These were measured from a pick-up on the dangling oscilloscope lead. It was first established that the mark/space ratio was directly proportional to the current drawn from the power source and it was the latter that was then used to register the power input into both the plasma generator and the discharge, usually between 0.15 and 1.0A, although this was not the actual current dissipated across the electrodes and within the discharge. An estimated 150 to 200 mW was coupled into the plasma.

For helium, argon and all other inert gases, discharges are an excellent source of their respective metastable states. For example, the metastable states of argon (Ar^{M}) are 4S, ³P_{2,0}. These are the first excited states above the ground state (¹S₀, the lowest energy state) in which argon can exist. These excited states lie at energies of 11.5 eV and 11.7 eV above the ground state.

Electron impact produces a number of excited states that decay to the metastable state, emitting light as they do so. The metastables are important as they have a very long lifetime (r). In the absence of quenching reactions the metastables are important in a Glow Discharge ion source as they contribute significantly to the ionisation of other atoms and molecules present in trace amounts within the gas (Penning ionisation).

In the presence of a small amount of water Ar^{M} reacts very quickly to give OH* (the A state) which fluoresces at 308 nm down to its ground state.

Ar³P_{2,0} + H₂O → OH* + H + Ar¹S₀

OH* → OH (ground state) + h 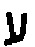 (λ= 308 nm)

It is likely that due to the prevalence of argon metastables in the afterglow this is the process that is dominating the plume reaction when the metastables meet water vapour. Small contributions from the Ar₂ (Rydberg state) reaction and direct electron impact on H₂O cannot however be ruled out as there will still be a contribution to the plume from the pulsed discharge travelling down the plume.

The following Examples were performed in a laboratory whose temperature varied between 19 to 23°C and whose relative humidity was in the order of 45%.

### Example 1

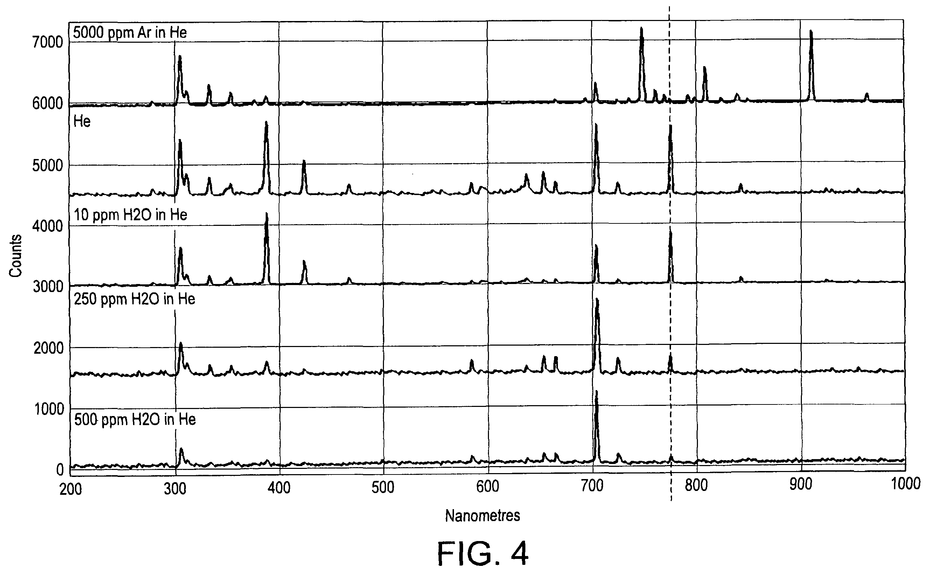

Referring to Figure 4, in this example the emitting excited species were counted by the optical emission spectrometer when the apparatus in Figure 18 was operated with (a) 5000 ppm Ar/He; (b) helium (comparative); (c) 10 ppm H₂O/He (comparative); (d) 250 ppm H₂O/He (comparative); and (e) 500 ppm H₂O/He (comparative). The emission spectra were recorded at a distance of 2mm from the plume exit. The flow rate in all cases is 0.5 L/min. In short, a high concentration of singlet oxygen atoms (777 nm) were detected in the helium plume by the emission spectrometer. However, this was almost eliminated by the inclusion of argon. It is believed that exposure of the plume to the atmosphere reduced the concentration of singlet oxygen atoms in the helium plume. As the water vapour concentration increases it can be seen that the singlet oxygen atom intensity peak significantly reduces. It can however also be seen that the excited OH intensity peak (308 nm) is also significantly reduced as the concentration of the water vapour increases.

### Example 2

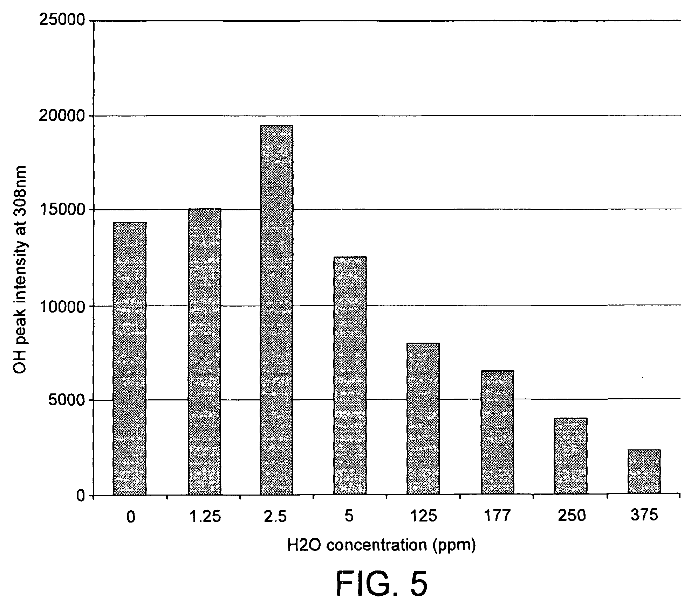

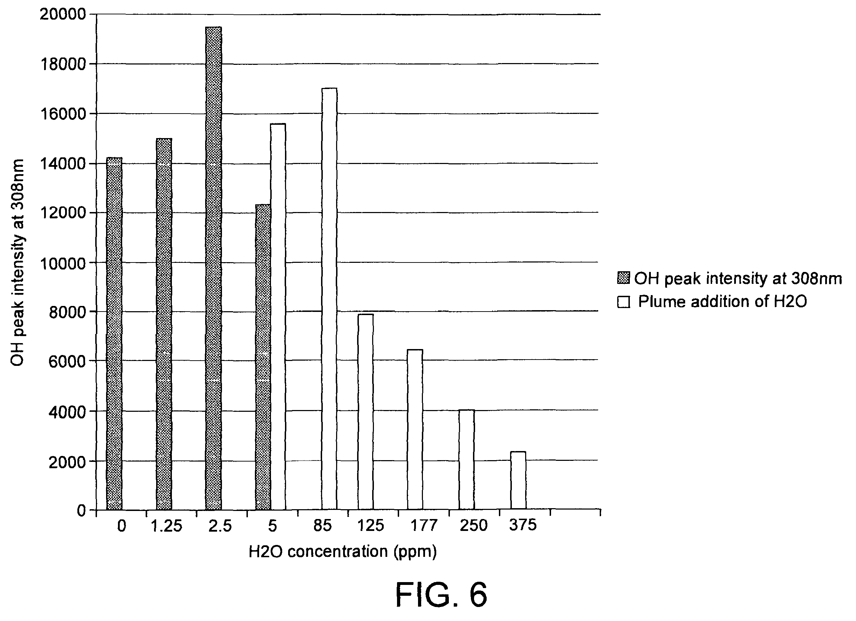

In this example the emitting excited species were counted by the optical emission spectrum when the apparatus shown in Figure 18 was operated with (a) a gas mixture of 5000 ppm Ar/He after being exposed to deliberately added water vapour within the plasma generator; and (b) a gas mixture of 5000 ppm Ar/He after exposing the plume to water vapour. Referring to Figures 5 and 6, the graphs illustrate the effect on the OH peak intensity at 308nm of the resulting plumes with respect to water concentration (ppm).

It can be seen from Figure 5 that there is no simple linear relationship between the population of emitted excited state species in the plume and the concentration of water vapour that the plasma has been exposed to. We attribute these results partly to a tendency we have found for the additive gas to quench the non-thermal plasma in the plasma generator. Once the maximum is reached, the plasma-quenching effect reduces the total number of ions and excited state species present in the plume. As helium has a particularly high ionisation energy, ions of the additive gas will be formed preferentially in the discharge.

Referring to Figure 6, it can be seen that the OH peak intensity at 308nm is significantly increased by exposing the plume rather than the plasma to water vapour. For example, it can be seen from Figures 5 and 6 that when the plasma is exposed to a water vapour concentration of 5 ppm the resulting OH peak has a relative intensity of just over 12000 counts per second. It can be seen from Figure 6 that by exposing the plume rather than the plasma to a water vapour concentration of 5 ppm the resulting relative OH peak intensity is about 15500 cps. Although there is no exact data for the OH peak intensity resulting from exposing the plasma to a water vapour concentration of 85 ppm, it can be seen from figure 5 that the OH peak intensity will be in the region of less than 10000. In contrast, Figure 6 shows that the OH peak intensity resulting from exposing the plume to a water vapour concentration of 85 ppm is approximately 17000.

The experiments therefore show that the OH peak intensity of the resulting plume is significantly increased by exposing the plume rather than the plasma to water vapour.

Even at zero deliberately added water vapour, there is a significant hydroxyl radical count indicating that the plume picks up water vapour from the atmosphere.

### Example 3

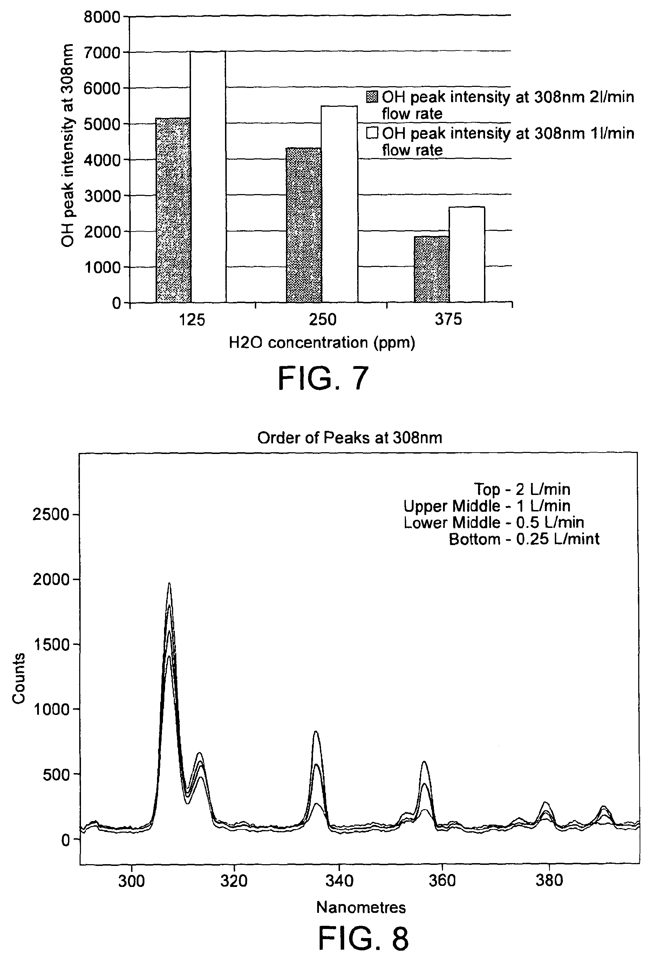

Referring to Figures 7, 8 and 9, in this example the excited emitting species were counted by the optical emission spectrometer when the apparatus in Figures 18 was operated at different flow rates using a gas mixture of 5000ppm Ar/He.

Referring to Figure 7, the plume was exposed to water vapour concentrations of 125 ppm, 250 ppm and 375 ppm. The experiments were carried out at two different flow rates (2L/min and 1 L/min). The results show that the OH peak intensity was higher at a lower flow rate of 1 Umin than at a flow rate of 2 L/min.

Figure 8 illustrates the effect of varying the flow rate on the resulting OH peak intensity of a gas mixture of 5000 ppm argon in helium. It can be seen from the peaks that varying the flow rate has only a modest effect on the excited OH concentration.

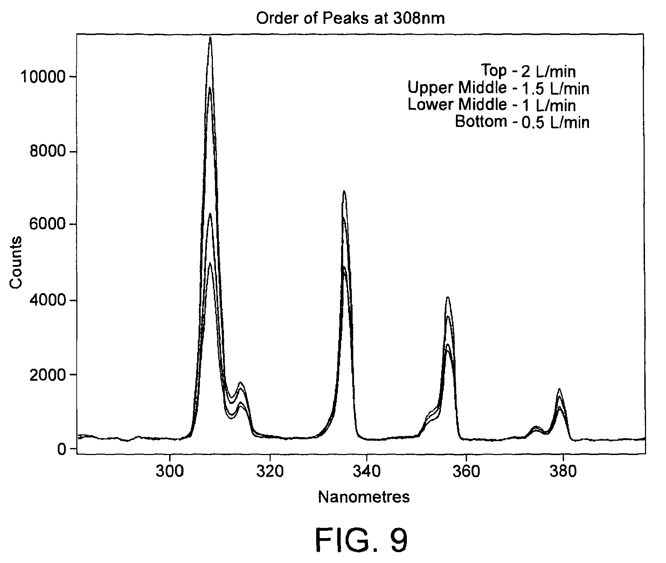

In contrast, Figure 9 illustrates the effect of varying the flow rate on the resulting OH concentration of a gas mixture of 5000 ppm argon/helium when the plume has been exposed to 330 ppm water vapour. The results show that increasing the flow rate has a much larger effect on the OH concentration.

### Example 4

In this example the excited state species were counted by the optical emission spectrometer when the apparatus in Figure 18 was operated by exposing the plume of a gas mixture of 5000 ppm Ar/He to different concentrations of water vapour.

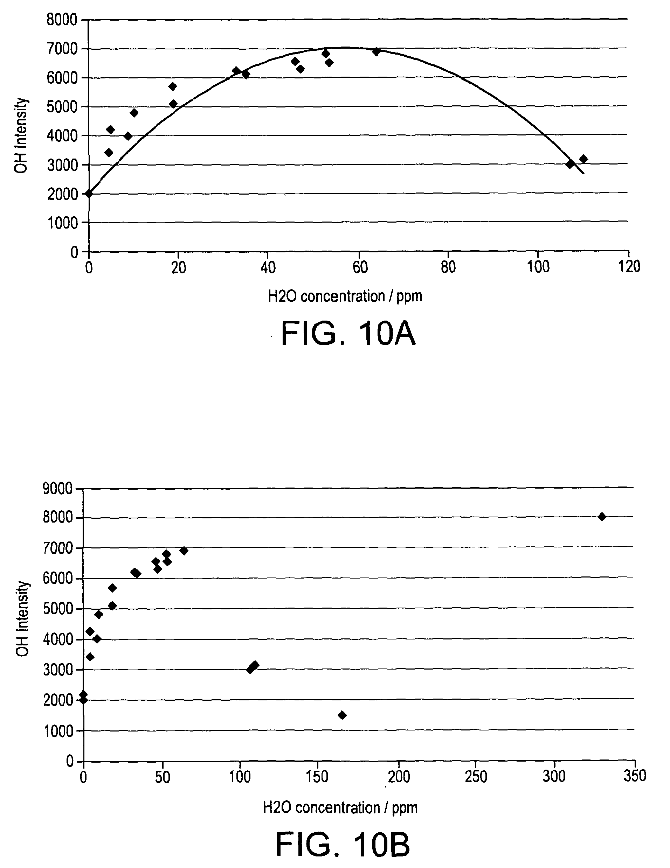

Referring to Figures 10A & 10B, it can be seen that there is no simple linear relationship between the OH peak intensity in the plume and the concentration of water vapour that the plasma has been exposed to.

It has been found that when the plasma of a gas mixture of 5000 ppm Ar/He is exposed to water vapour the maximum OH peak intensity is reached when the plasma is exposed to a water vapour concentration of 2.5 ppm.

Referring to figures 10A and 10B, it can be seen that the maximum OH peak intensity is reached when the plume is exposed to a water vapour concentration of 50 ppm (using 250 ppm H₂O/He).

It has been found that the OH peak intensity when exposing the plume to water vapour is 30-50% greater than the OH peak intensity resulting from exposure of the plasma to water vapour.

We have found that when the highest OH peak intensity was obtained using addition of 330 ppm H₂O/He to 5000 ppm Ar/He, the plume temperature was 34°C. We have found that when the highest OH peak intensity was obtained using addition of 450 ppm H₂O/He to 5000 ppm Ar/He the plume temperature was 64°C and produced significant levels of NO. We have also found that when the highest OH peak intensity was obtained at 85-100 ppm H₂O by using plasma addition of 10 ppm H₂O/He to 5000 ppm Ar/He, the plume temperature was 37°C. We have found that when the highest OH peak intensity was obtained at 85-100 ppm H₂O using plasma addition of 250 ppm H₂O/He to 5000 ppm Ar/He, the plume temperature was 37°C.

A further experiment was carried out, exposing the plume (5000 ppm Ar/He) to 500 ppm oxygen/helium. The results showed a significant reduction in OH peak intensity (308 nm emission) at all concentrations and significant singlet oxygen peak intensity at 777 nm.

It can therefore be seen that the method of the invention provides plumes with increased excited OH peak intensity. The resulting plumes will therefore probably have improved bactericidal activity. Further, it has been shown that the resulting plumes have reduced singlet oxygen peak intensity, and therefore probably reduced ground state atom concentration and therefore the method of the invention has the advantage that the resulting plume may form a reduced amount of ozone which will help to reduce negative side effects for the patient.

### Example 5

In this example the temperature of the plume produced from the apparatus of Figures 2, 3 and 18 was measured at a distance of 2mm from the plume exit for a number of gas mixtures of Ar and He containing a varying concentration of argon.

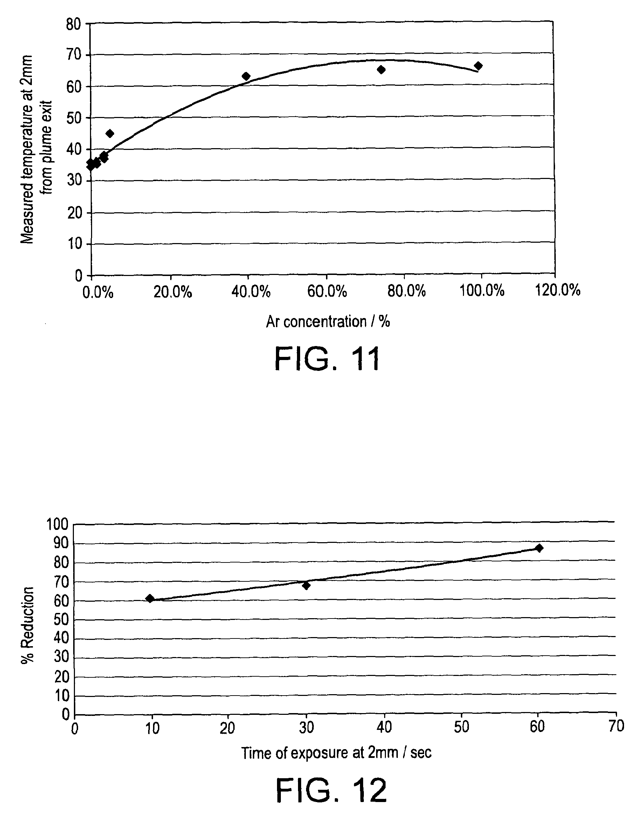

Referring to Figure 11, it can be seen that the temperature of the plume is dependent on the amount of argon present within the gas mixture. The temperature of the plume increases as the amount of argon in the gas mixture increases.

The plasma generator preferably has a gas outlet temperature of from 5°C to 42°C, for example from 10°C to 40°C. Higher gas temperatures are generally unsuitable for oral treatments and may damage the mouth or teeth if sustained for too long a period. Temperatures lower than 5°C may be found uncomfortable by the person undergoing the treatment and in any event are difficult to achieve without unnecessary cooling of the gas mixture.

As shown in Figure 11, in order to be suitable for oral temperatures (therefore having an outlet plume temperature of 42°C or less) the concentration of argon in the gas mixture is preferably less than about 4% by volume. Initial experiments with helium-neon which are exposed to water vapour, indicate that tooth whitening/anti-bacterial results superior to those described herein in relation to helium-argon mixtures may be obtained.

### Example 6

A non-thermal gaseous plasma was created using an apparatus of the kind described with reference to Figure 18 from the gas stream (flow rate 0.5l/min) formed of 5000 ppm argon/helium. The partially-ionised gas exiting the plasma generator was applied as a plume to colonies of *Streptoccoci mutans* (NCTC-10919). Experiments were performed for exposure times of 10s, 30s and 60s.

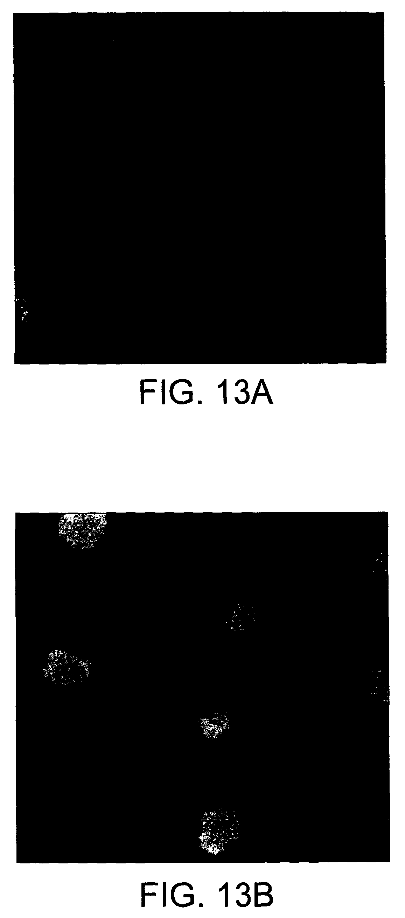

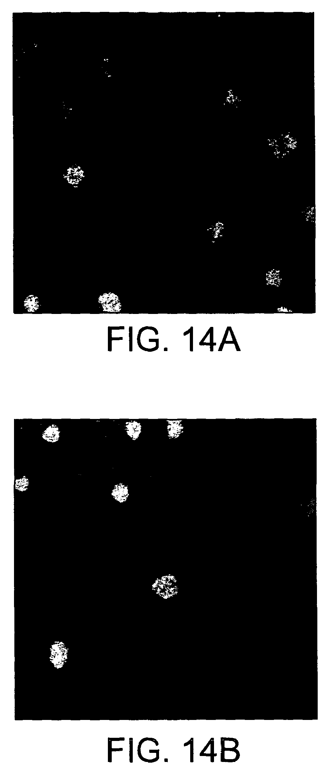

Referring to figure 12, the graph shows that all these exposure times show destruction of cells. Increasing the exposure time to the plume increases the % reduction in cells. Referring to figures 13 and 14, the colonies were exposed to the plume for 60s.

Figures 13 and 14 shows that eradicated cells (figures 13A and 14A) are readily distinguishable from healthy cells (figures 13B and 14B). Eradicated cells are shown only as amorphous cellular debris. Healthy bacteria (figures 13B and 14B) give a Gram stain that is blue and have well defined shaped. Damaged bacteria give a Gram stain that is pink. The photographs were taken at x100 magnification.

### Example 7

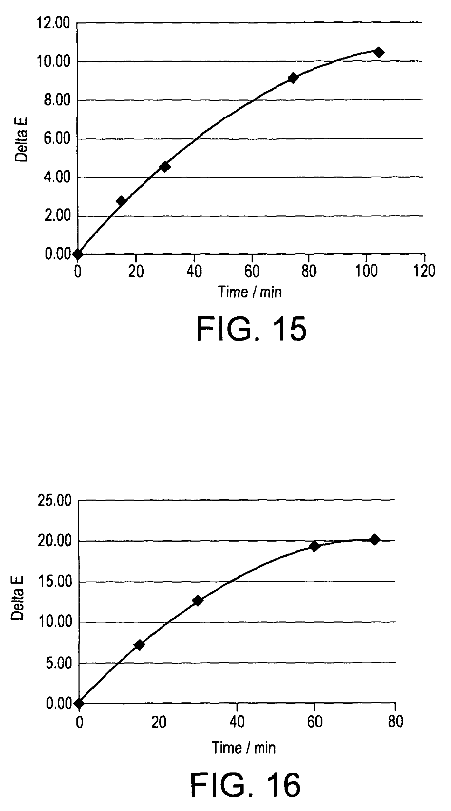

Referring to Figures 15 and 16, experiments were carried out to investigate the whitening effect over time of the plume resulting from the partial ionisation of (a) a gas mixture of 330ppm H₂O/Ar diluted with He (∼1:5) (Figure 15) and (b) a gas mixture of 5000 ppm Ar/He (Figure 16) at a distance of 2mm. Both of these figures show that that whitening effect initially increases with time. Delta E represents an increase in whiteness. The higher the delta E value the greater the whitening effect. After a certain period the whitening effect (delta E) slows down until a maximum whitening effect has been reached.

In Figure 15 it shows that the whitening effect (delta E) of the plume of the gas mixture 330 ppm H₂O/Ar diluted with He (∼1:5) increases steadily for 80 minutes. At this point the whitening effect slows down to reach a maximum (delta E approx. 10) at around 100 minutes. In contrast, in figure 16 the delta E of the gas mixture of 5000 ppm Ar/He increases steadily for the first 50 minutes. At this point the whitening effect of the plume slows down to reach a maximum (delta E of 20) at around 80 minutes.

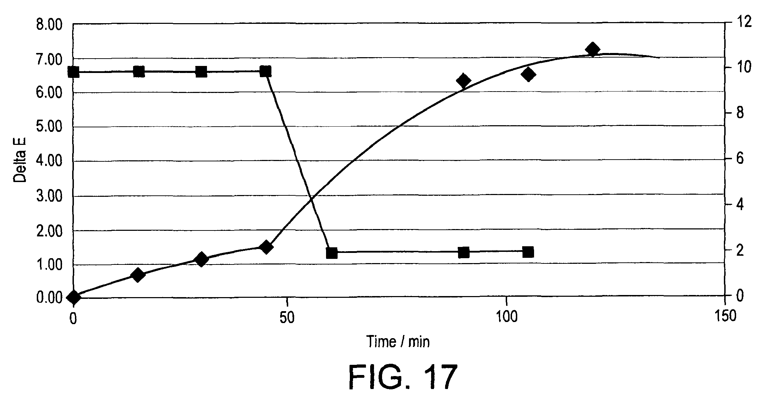

Figure 17 illustrates the whitening effect (delta E) over time of the plume resulting from the partial ionisation of a gas mixture of 330ppm H₂O/Ar diluted with He (∼1:5) at a distance of 10mm for 45 minutes and a distance of 2mm thereafter. The blue line illustrates the whitening effect (delta E) over time. The brown line illustrates the distance of the plume.

### Example 8

The effect on the number of colony forming units (CFU) of Streptococcus mutans present after carrying out teeth whitening using different plasma conditions is shown in Table 1. The bacteriological counts are log₁₀ transformed prior to statistical analysis so that variances are homogenized. The total colony forming units (CFU) obtained after exposure to each of the plasma conditions are illustrated in table 1.

Gas mixtures which include water vapour are taken to mean gas mixtures which are exposed to water vapour down stream of the generator.

### Streptococcus mutans NCTC 10919

**Table 1**

| **Date and plasma conditions (Distance 2mm)** | **Exposure (sec)** | **CFU/ml** | **%Reduction** |
|---|---|---|---|
| 17/8) 5,000ppm Ar in He - 0.5 L/min | 60 | 4.7 x 10⁷ | 87 |
| 23/8) 5.000ppm Ar in He - 0.5 L/min | 10 | 3.8 x 10⁵ | 62 |
| 23/8) 5,000ppm Ar in He - 0.5 L/min | 30 | 3.2 x 10⁵ | 68 |
| 24/8 ArH₂O 450ppm 0.1L/min He 0.6L/min | 10 | 3.8 x 10⁶ | 34 |
| 24/8 ArH₂O 450ppm 0.1L/min He 0.6L/min | 30 | 3 x 10⁶ | 48 |
| 31/8) 2.5ppm H₂O in 3750ppm Ar and 250,000ppmHe | 10 | 1.6x 10⁶ | 48.15 |
| 31/8) 2.5ppm H₂O in 3750ppm Ar and 250,000ppmHe | 30 | 6 x 10⁵ | 44 |
| 2/9) 90ppm H₂O in 200,000ppmAr and 800,000ppm He | 10 | 5 x 10⁵ | 51 |
| 2/9) 90ppm H₂O in 200,000ppmAr and 800,000ppm He | 30 | 1.02 x 10⁵ | 90 |

| Streptococcus sanguinis NCTC 10904 | | | |
|---|---|---|---|
| **Date and plasma conditions** | **Exposure (sec)** | **CFU/ml** | **%Reduction** |
| 17/8) 5,000ppm Ar in He - 0.5 L/min | 60 | 2.12 x 10⁶ | 96 |
| 23/8) 5,000ppm Ar in He - 0.5 L/min | 10 | 3 x 10⁵ | 96 |
| 23/8) 5,000ppm Ar in He - 0.5 L/min | 30 | 1 x 10⁵ | 99 |
| 24/8 ArH₂O 450ppm 0.1L/min He 0.6L/min | 10 | 6 x 10⁵ | 92 |
| 24/8 ArH₂O 450ppm 0.1L/min He 0.6L/min | 30 | 1.6 x 10⁵ | 98 |
| 31/8) 2.5ppm H₂O in 3750ppm Ar and 250,000ppmHe | 10 | 1.66 x 10⁵ | 79 |
| 31/8) 2.5ppm H₂O in 3750ppm Ar and 250.000ppmHe | 30 | 3.2 x 10⁴ | 96 |
| 2/9) 9ppm H₂O in 2%Ar and 98%He | 10 | 2.8 x 10⁴ | 90 |
| 2/9) 90ppm H₂O in 2% Ar and 98% He | 30 | 4.8 x 10⁴ | 83 |

## Claims

1. A method of making an active gas, comprising generating within a plasma generator (36) a glow discharge, non-thermal, plasma in a flow of gas mixture comprising a carrier gas and a more readily ionisable gas to form an active gas mixture, and exposing the active gas mixture to a source (37) of water vapourdownstream from the generator (36) so as to form the active gas, wherein
(a) the carrier gas is helium;
(b) the more readily ionisable gas is at least one noble gas selected from the group consisting of Argon, Neon, Krypton, and Xenon;
(c) the gas mixture includes up to 40% by volume of the more readily ionisable gas;
(d) the active gas mixture is ejected at a temperature in the range from 55°C to 42°C, and
the active gas mixture flows from the generator (36) through a passage and is ejected from the passage into a standing gaseous atmosphere,
the standing gaseous atmosphere contains water vapour from the source (37) and the flow of the active gas mixture through the gaseous atmosphere thereby exposes the active gas mixture to the water vapour (37), so as to form the active gas andthe standing gaseous atmosphere is ambient air.

2. A method according to claim 1, wherein the pressure of the gas mixture is in the range of from 0.5 to 2 bar.

3. A method according to claim 2, wherein the pressure of the gas mixture in the generator (36) is in the range from 0.9 to 1.1 bar.

4. A method according to claim 1, in which the concentration of the more readily ionisable gas is less than or equal to 4% by volume, the more readily ionisable gas being argon.

5. A method according to any one of the preceding claims, in which the more readily ionisable gas is argon.

6. A method according to any one of the preceding claims, wherein the active gas has bactericidal activity.

7. A method according to any one of the preceding claims, wherein the active gas has whitening activity.

8. A method according to claim 6, wherein the active gas has oral bactericidal activity.

9. A method as claimed in claim 7, wherein the whitening activity is tooth whitening activity.

10. Use of an active gas prepared according to the method as claimed in any one of claims 1 to 9 for bactericidal and/or whitening purposes.

11. A handheld oral hygiene device comprising an apparatus for making an active gas in accordance with the method of any one of claims 1 to 9 and a gas compartment for containing a mixture of the carrier gas and the more readily ionisable gas, said apparatus comprising a housing containing the plasma generator, at least one cathode and at least one anode, and a voltage signal generator operatively associated with the anode and the cathode to produce a voltage signal suitable for the generation of non-thermal plasma.

12. A method of stain removal, comprising:
i. making an active gas in accordance with the method of any one of claims 1 to 9 ; and
ii. directing a flow of the active gas at an article.

13. A method of bacteria removal comprising:
i. making an active gas in accordance with the method of any one of claims 1 to 9; and
ii. directing a flow of the active gas at an article.

## Patentansprüche

1. Verfahren zum Herstellen eines aktiven Gases, umfassend Generieren eines nicht-thermischen Glimmentladungsplasmas in einem Strom einer Gasmischung, die ein Trägergas und ein leichter ionisierbares Gas umfasst, im Inneren eines Plasmagenerators (36), um eine aktive Gasmischung zu bilden, und Aussetzen der aktiven Gasmischung einer Quelle (37) für Wasserdampf nachgeordnet zu dem Generator (36), um so das aktive Gas zu bilden, wobei
**(a)** das Trägergas Helium ist;
**(b)** das leichter ionisierbare Gas mindestens ein Edelgas ausgewählt aus der Gruppe bestehend aus Argon, Neon, Krypton und Xenon ist;
**(c)** die Gasmischung bis zu 40 Vol.% des leichter ionisierbaren Gases einschließt;
**(d)** die aktive Gasmischung bei einer Temperatur im Bereich von 5 °C bis 42 °C ausgestoßen wird, und
die aktive Gasmischung von dem Generator (36) durch einen Durchgang fließt und aus dem Durchgang in eine unbewegte gasförmige Atmosphäre ausgestoßen wird,
wobei die unbewegte gasförmige Atmosphäre Wasserdampf aus der Quelle (37) enthält und der Strom der aktiven Gasmischung durch die gasförmige Atmosphäre die aktive Gasmischung dadurch dem Wasserdampf (37) aussetzt, um so das aktive Gas zu bilden, und wobei die unbewegte gasförmige Atmosphäre Umgebungsluft ist.

2. Verfahren nach Anspruch 1, wobei der Druck der Gasmischung im Bereich von 0,5 bis 2 bar liegt.

3. Verfahren nach Anspruch 2, wobei der Druck der Gasmischung im Generator (36) im Bereich von 0,9 bis 1,1 bar liegt.

4. Verfahren nach Anspruch 1, wobei die Konzentration des leichter ionisierbaren Gases kleiner als oder gleich 4 Vol.% ist, wobei das leichter ionisierbare Gas Argon ist.

5. Verfahren nach einem der vorhergehenden Ansprüche, wobei das leichter ionisierbare Gas Argon ist.

6. Verfahren nach einem der vorhergehenden Ansprüche, wobei das aktive Gas bakterizide Aktivität hat.

7. Verfahren nach einem der vorhergehenden Ansprüche, wobei das aktive Gas Bleichaktivität hat.

8. Verfahren nach Anspruch 6, wobei das aktive Gas oral bakterizide Aktivität hat.

9. Verfahren nach Anspruch 7, wobei die Bleichaktivität Zahnbleichaktivität ist.

10. Verwendung eines aktiven Gases, das nach dem Verfahren gemäß einem der Ansprüche 1 bis 9 hergestellt ist, zu bakteriziden und/oder Bleichzwecken.

11. Handgeführte Mundhygienevorrichtung, umfassend einen Apparat zum Herstellen eines aktiven Gases gemäß dem Verfahren nach einem der Ansprüche 1 bis 9 und einen Gasraum zur Aufnahme einer Mischung des Trägergases und des leichter ionisierbaren Gases, wobei der Apparat ein Gehäuse, enthaltend den Plasmagenerator, mindestens eine Kathode und mindestens eine Anode und einen Spannungssignalgenerator umfasst, der in Wirkbeziehung mit der Anode und der Kathode verbunden ist, um ein Spannungssignal zu produzieren, das zum Generieren von nicht-thermischem Plasma geeignet ist.

12. Verfahren zur Entfernung von Verfärbungen, umfassend:
i. Herstellen eines aktiven Gases gemäß dem Verfahren nach einem der Ansprüche 1 bis 9; und
ii. Leiten eines Stroms des aktiven Gases zu einem Gegenstand.

13. Verfahren zur Bakterienentfernung, umfassend:
i. Herstellen eines aktiven Gases gemäß dem Verfahren nach einem der Ansprüche 1 bis 9; und
**ii.** Leiten eines Stroms des aktiven Gases zu
einem Gegenstand.

## Revendications

1. Procédé de fabrication d'un gaz actif, comprenant la production, à l'intérieur d'un générateur de plasma (36), d'un plasma de décharge luminescente, non thermique dans un écoulement de mélange gazeux comprenant un gaz vecteur et un gaz plus facilement ionisable pour former un mélange gazeux actif, et l'exposition du mélange gazeux actif à une source (37) de vapeur d'eau en aval du générateur (36) de manière à former le gaz actif, dans lequel
(a) le gaz vecteur est l'hélium ;
(b) le gaz plus facilement ionisable est au moins un gaz rare choisi dans le groupe constitué par l'argon, le néon, le krypton, et le xénon ;
(c) le mélange gazeux comporte jusqu'à 40 % en volume du gaz plus facilement ionisable ;
(d) le mélange gazeux actif est éjecté à une température dans la gamme de 5 °C à 42 °C, et
le mélange gazeux actif s'écoule depuis le générateur (36) à travers un passage et est éjecté depuis le passage dans une atmosphère gazeuse d'attente,
l'atmosphère gazeuse d'attente contient de la vapeur d'eau provenant de la source (37) et l'écoulement du mélange gazeux actif à travers l'atmosphère gazeuse expose ainsi le mélange gazeux actif à la vapeur d'eau (37) de manière à former le gaz actif, et l'atmosphère gazeuse d'attente est l'air ambiant.

2. Procédé selon la revendication 1, dans lequel la pression du mélange gazeux se situe dans la gamme de 0,5 à 2 bar.

3. Procédé selon la revendication 2, dans lequel la pression du mélange gazeux dans le générateur (36) se situe dans la gamme de 0,9 à 1,1 bar.

4. Procédé selon la revendication 1, dans lequel la concentration du gaz plus facilement ionisable est inférieure ou égale à 4 % en volume, le gaz plus facilement ionisable étant l'argon.

5. Procédé selon l'une quelconque des revendications précédentes, dans lequel le gaz plus facilement ionisable est l'argon.

6. Procédé selon l'une quelconque des revendications précédentes, dans lequel le gaz actif a une activité bactéricide.

7. Procédé selon l'une quelconque des revendications précédentes, dans lequel le gaz actif a une activité de blanchiment.

8. Procédé selon la revendication 6, dans lequel le gaz actif a une activité bactéricide buccale.

9. Procédé selon la revendication 7, dans lequel l'activité de blanchiment est une activité de blanchiment des dents.

10. Utilisation d'un gaz actif préparé conformément au procédé selon l'une quelconque des revendications 1 à 9 à des fins bactéricides et/ou de blanchiment.

11. Dispositif d'hygiène buccale à main comprenant un appareil destiné à fabriquer un gaz actif conformément au procédé de l'une quelconque des revendications 1 à 9 et un compartiment à gaz destiné à contenir un mélange du gaz vecteur et du gaz plus facilement ionisable, ledit appareil comprenant un boîtier contenant le générateur de plasma, au moins une cathode et au moins une anode, et un générateur de signal de tension fonctionnellement associé à l'anode et la cathode pour générer un signal de tension approprié pour la production d'un plasma non thermique.

12. Procédé de retrait de taches, comprenant :
i. la fabrication d'un gaz actif conformément au procédé de l'une quelconque des revendications 1 à 9 ; et
ii. l'acheminement d'un écoulement du gaz actif au niveau d'un article.

13. Procédé d'élimination de bactéries comprenant :
i. la fabrication d'un gaz actif conformément au procédé de l'une quelconque des revendications 1 à 9 ; et
ii. l'acheminement d'un écoulement du gaz actif au niveau d'un article.
